# EUROPÄISCHE PATENTSCHRIFT

(11) **EP 1 299 027 B1**
(45) Veröffentlichungstag und Bekanntmachung des Hinweises auf die Patenterteilung: **19.04.2006**
(21) Anmeldenummer: 01953841.2
(22) Anmeldetag: 06.07.2001
(51) Int. Cl.: A61B 1/267

(54) **LARYNGOSKOP**
LARYNGOSCOPE
LARYNGOSCOPE

(30) Priorität: 07.07.2000 DE 10032716
(43) Veröffentlichungstag der Anmeldung: 09.04.2003
(73) Patentinhaber: Kellner, Peter, 36269 Phillippsthal (DE)
(72) Erfinder: KELLNER, Peter, 36269 Phillippsthal (DE); GOOS, Christoph, 24105 Kiel (DE)
(74) Vertreter: Liedtke, Klaus
(86) Internationale Anmeldenummer: PCT/DE2001/002455
(87) Internationale Veröffentlichungsnummer: WO 2002/003851

(56) Entgegenhaltungen:
- EP-A- 0 125 687
- EP-A- 0 339 541
- EP-A- 0 901 772
- DE-A- 3 119 725
- DE-U- 9 109 163
- FR-A- 2 381 528
- US-A- 5 438 976

## Beschreibung

Die Erfindung betrifft ein Laryngoskop zur Untersuchung des Rachenraumes mit einem Tubusgriff, einer Lichtquelle und einem Intubationsspatel, der ein gebogenes Unterteil mit einer seitlich daran angebrachten Führungsleiste aufweist, die sich am distalen Ende verjüngt. Die Führungsleiste beinhaltet lichtleitende Fasern, die an der sich zum distalen Ende verjüngenden Leiste austreten.

Laryngoskope werden in der Anästhesie verwendet und dienen zur Platzierung eines Beatmungstubus in der Luftröhre eines muskelrelaxierten Patienten zwecks Beatmung während einer Operation, auf der Intensivstation oder bei einem Notfall.

Laryngoskope bestehen aus einem Handgriff und einem daran rechtwinklig angeordneten Spatel zum Niederdrücken der Zunge des Patienten. Im distalen Bereich des Spatels ist eine Lichtquelle angebracht, mit der der Rachenraum des Patienten ausgeleuchtet werden kann. Nach dem Einführen des Spatels wird an dem selbigen der Beatmungstubus entlang geführt und in die Luftröhre eingeführt. Die Anwendung bedarf viel Erfahrung und Können.

Bei den bekannten Laryngoskopen besteht der Spatel aus einer gebogenen Platte, die in Längsrichtung eine Führungsleiste aufweist, an welcher der Tubus entlang geführt wird. Eine solche Anordnung ist beispielsweise in DE 33 17 831 Al beschrieben.
Nachteilig ist dabei aber die zum proximalen Bereich des Spatels zunehmende Höhe der Führungsleiste, die deshalb eine hohe Gefahr zur Beschädigung der oberen Schneidezähne bei falscher oder ungeschickter Benutzung des Laryngoskops in sich birgt.

In US 4,112,933 ist eine Laryngoskopausführung beschrieben, dessen Spatel eine bogenförmig gewölbte Form aufweist.. Nachteilig sind hierbei insbesondere die Verletzungsgefahr und die komplizierte Herstellung.

Mit dem in DE 91 09 163.2 Ul beschriebenen Laryngoskopspatel wurde versucht, die Gefahr der Zahnverletzungen dadurch zu verhindern, dass am proximalen Teil des Spatels die Führungsleiste ganz weggelassen wird. Ferner besteht bei diesem Laryngoskop der distale Bereich des Spatels zur Anpassung an anatomische Erfordernisse aus einem formbaren Material.

Nachteilig ist hierbei die schwierige Herstellung des Spatels. Es erscheint auch fraglich, ob die Stellung der Spatelspitze beim Einsatz in der vorgebogenen Stellung verbleibt und ob nicht mit deren Verbiegung die Sicht auf Stimmbänder und Luftröhre wieder verlegt würde. Ungünstig ist außerdem, dass durch die schmale Ausführung des Spatels am proximalen Ende die Zunge des Patienten nicht ausreichend abgehalten werden kann, so dass für den Behandler eine erhebliche Sichteinschränkung auftritt.

Ferner ist nach DE 31 19 725 A1 ein Laryngoskop bekannt, dessen Spatel ein etwa L- oder Z-förmiges Profil aufweist. Um eine einfache Herstellung zu ermöglichen, besteht der Spatel bei diesem Laryngoskop aus zwei übereinandergreifend angesetzten und miteinander verlöteten L-förmigen Längsprofilen.

Der Erfindung liegt die Aufgabe zugrunde, ein Laryngoskop zu schaffen, das einfach herstellbar ist und bei dem die Verletzungsgefahr im Zahnbereich weitgehend vermieden wird.

Erfindungsgemäß wird die Aufgabe mit einem Laryngoskop gelöst, dass die in Anspruch 1 angegebenen Merkmale aufweist.

Vorteilhafte Ausgestaltungen sind in den Unteransprüchen angegeben.

Bei dem erfindungsgemäßen Laryngoskop weist der Intubationsspatel eine verkürzte Führungsleiste und am Ende eine flache, breite Form auf Der senkrechte Schenkel, die Führungsleiste, ist am proximalen Ende bis zur Höhe 0 verringert und der waagerechte Schenkel ist in voller Breite bis zum proximalen Ende ausgeführt. Damit kann eine stabile Ausführung des Spatels erreicht werden, die gleichzeitig die Gefahr von Verletzungen, insbesondere von Beschädigungen der Zähne des Patienten, stark mindert.

Am Handgriff des Laryngoskops wird ein Ring mit Hilfe eines kleinen Hebels positioniert. Der Hebel ist dabei Betätigungselement der Klemmvorrichtung und wirkt gleichzeitig als Widerlager, mit dem eine sichere Abstützung der Finger des Behandlers ermöglicht wird, so dass das Laryngoskop gezogen werden kann, ohne dass ein Hebeln erfolgt und damit die Verletzungsgefahr für die Zähne des Patienten vermieden wird.
Am Ende des Intubationsspatels ist eine Kaltlichtlampe angebracht, die über Glasfasern versorgt wird, welche sich neben der Führungsleiste befinden und von dieser abgedeckt werden.

Die Erfindung wird im Folgenden an einem Ausführungsbeispiel näher erläutert. In den zugehörigen Zeichnungen zeigen:
Figur 1 den Handgriff des Laryngoskops mit einem Ring und daran angebrachten Hebel in perspektivischer Darstellung,
Figur 2 die Seitenansicht der in Figur 1 erläuterten Anordnung,
Figur 3 die zugehörige Draufsicht,
Figur 4 eine Seitenansicht des Intubationsspatels,
Figur 5 die zugehörige Rückansicht,
Figur 6 die zugehörige Draufsicht,
Figur 7 eine Ansicht von der Gegenseite,
Figur 8 eine Ausführung mit geradem Unterteil im proximalen Bereich und
Figur 9 eine perspektivische Ansicht des Spatels.

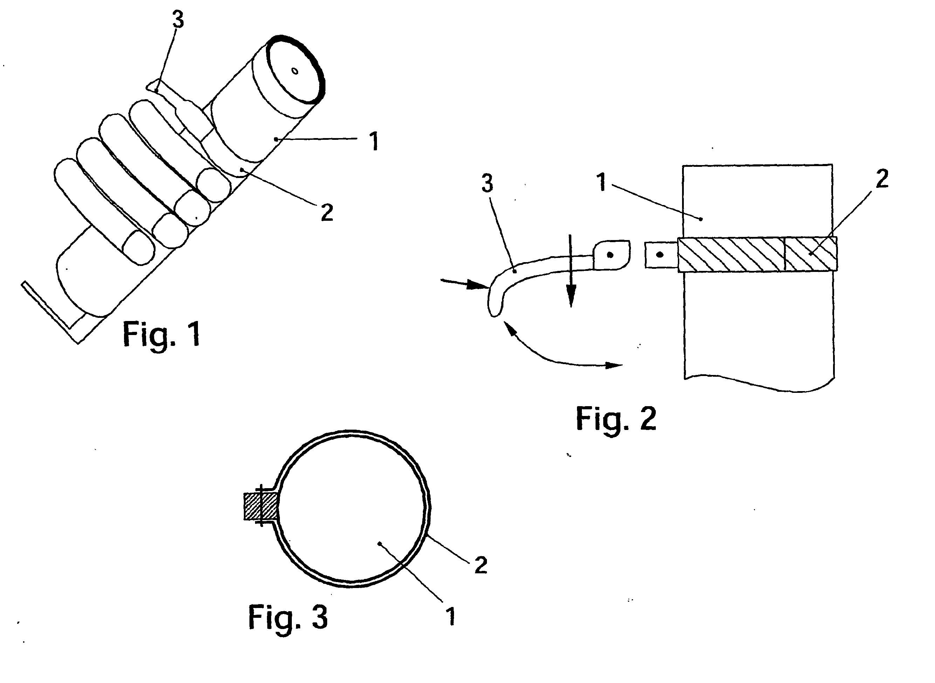

Figur 1 erläutert die Funktionsweise des Widerlagers an einen herkömmlichen Handgriff 1. Am Handgriff 1 des Laryngoskops befindet sich der Ring 2, der mit dem kleinen Hebel 3, zusammengehalten wird. Der als Widerlager wirkende Hebel 3 ist im Ring 2 gelagert. Die Lagerung ist exzentrisch ausgebildet, so dass in der dargestellten Position der Ring 2 geklemmt ist und die Hand des Behandlers beim Ziehen am Handgriff 1 nicht wegrutschen kann.

Wie aus den Figuren 2 und 3 ersichtlich ist, wird der Ring 2 über den Handgriff 1 des Laryngoskops gesteckt. Um den Ring 2 an die richtige Stelle zu schieben, wird der Hebel 3 heruntergeklappt. Nach erfolgter Positionierung wird er dann aufgerichtet und bewirkt die Arretierung des Rings 2. Diese Ausführung ermöglicht es, den Ring 2 auf jede Handbreite einzustellen. Vorteilhaft ist dabei, dass der Ring auf jedes schon vorhandene Laryngoskop nachgerüstet werden kann.

Durch das von dem Hebel 3 gebildete Widerlager lässt sich das Laryngoskop einfacher am Handgriff ziehen und die gelegentlich falsche Hebelbewegung und damit die Gefahr von Verletzungen entfällt.

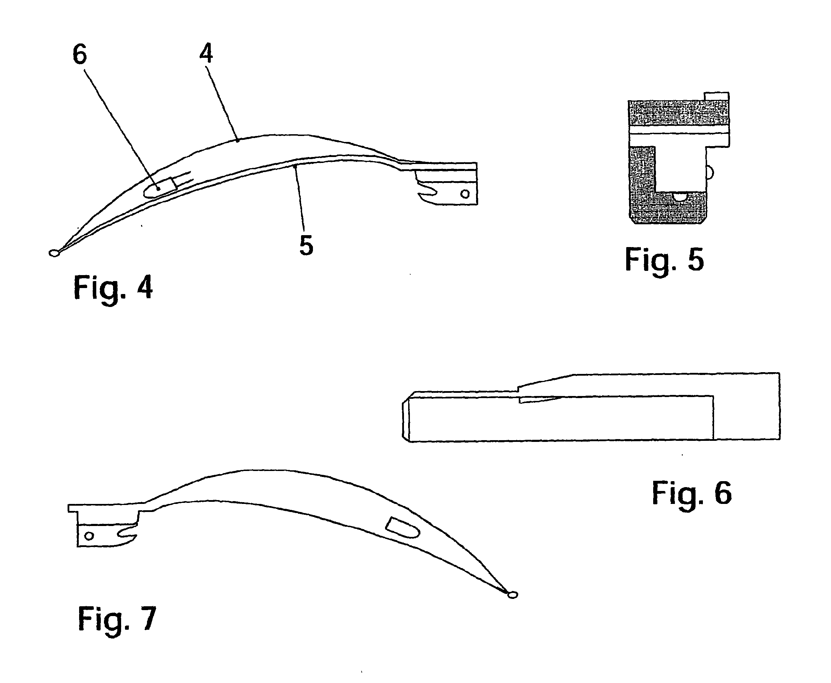

In den Figuren 4 bis 7 ist der Intubationsspatel in vier Ansichten dargestellt. Er besteht aus einem flachen Unterteil 5, einer Führungsleiste 4, einer kleinen Kaltlichtlampe 6 und einem Kanal für die Führung von Glasfasern, in denen Licht zur Kaltlichtlampe 6 geführt wird. Bei einer vorteilhaften Ausführung erstreckt sich die Führungsleiste 4 von der Mitte des Intubationsspatels bis zu dessen distalem Ende. Der vordere Teil ist fast so flach wie ein normaler Zungenspatel ausgeführt, behält aber die volle Spatelbreite bis zum proximalen Ende bei.
Durch diese Gestaltung wird die Gefahr, die oberen Schneidezähne des Patienten zu berühren, deutlich herabgesetzt. Der Kanal für die Glasfasern des Lichtleitkabels kann dabei trotzdem noch seitlich der Führungsleiste 4 positioniert werden. Die Kaltlichtlampe 6 befindet sich in der Nähe des proximalen Spatelendes. Sie ermöglicht eine gute Ausleuchtung des Rachenraumes während der Intubation.

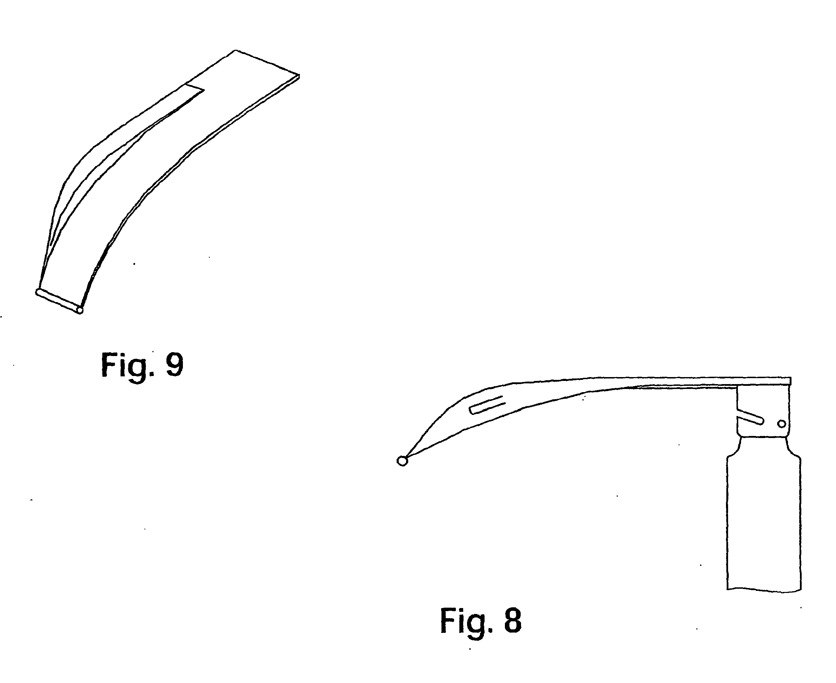

In Figur 8 ist eine Ausführungsform dargestellt, bei der das Unterteil im proximalen Drittel des Spatels gerade, ohne Krümmung ausgebildet ist. Diese Gestaltung erleichtert zusätzlich die Intubation, da sie die Zunge des Patienten noch mehr aus dem Sichtfeld des Behandlers hält. Aufgrund der gegenüber Erwachsenen anderen Anatomie des Kinderrachens bietet diese Streckung gleichfalls Vorteile bei der Anwendung an Kindern.

Figur 9 zeigt eine perspektivische Darstellung eines erfindungsgemäßen Spatels.

Der erfindungsgemäße flachere und breitere Intubationsspatel und der Ring am Griff des Laryngoskops ermöglichen eine sicherere Handhabung und einen universellen Einsatz. Insbesondere für Ungeübte, in Notfallsituationen und bei der Intubation von Patienten mit fliehendem Kinn und von Kindern, die erfahrungsgemäß kleinere Mundöffnungen haben, ist die Benutzung dieses Intubationsspatels besonders vorteilhaft, da die Gefahr der Verletzung der oberen Schneidezähne deutlich reduziert wird.

### Bezugszeichenliste

- 1: Tubusgriff
- 2: Ring
- 3: Hebel
- 4: Führungsleiste
- 5: flaches Unterteil
- 6: Kaltlichtlampe

## Patentansprüche

1. Laryngoskop zur Untersuchung des Rachenraumes mit einem Tubusgriff (1), einer Lichtquelle (6) und einem Intubationsspatel, der ein gebogenes Unterteil mit einer seitlich daran angebrachten Führungsleiste aufweist, die sich am distalen Ende verjüngt, wobei die Führungsleiste am proximalen Ende eine Verjüngung aufweist, die sich mindestens über das letzte Drittel der Spatellänge erstreckt und bis zur Höhe Null geführt wird und dass der waagerechte Schenkel in voller Breite bis zum proximalen Ende geführt ist, wobei das Unterteil im proximalen Ende gerade ausgebildet ist, **dadurch gekennzeichnet, dass** am Intubationsspatel eine Kaltlichtlampe (6) und ein Kanal angeordnet sind, wobei sich in dem Kanal Lichtleitfasern befinden und dass an dem Tubusgriff (1) ein längsverschiebbarer Ring (2) mit einem kleinen Hebel (3) angeordnet ist, der am Tubusgriff (1) in beliebiger Lage positionierbar und klemmbar ist.

2. Laryngoskop nach Anspruch 1, **dadurch gekennzeichnet, dass** das Unterteil am proximalen Ende durch Vergrößern der Wandstärke und/oder durch Anbringen einer Sicke versteift ist.

## Claims

1. A laryngoscope for examining the throat area, having a tube-shaped handle (1), a light source (6) and an intubation blade which has a curved bottom part with a guiding gib laterally attached to it, the guiding gib narrowing at a distal end and exhibiting a tapering at a proximal end, wherein the tapering extends at least across the last third of the blade length and is led to a height of zero, and wherein the horizontal piece is led to the proximal end with full width, wherein the bottom part is formed straight at the proximal end, **characterized in that** a cold light lamp (6) and a channel are arranged at the intubation blade, wherein fibre optical waveguides are located in the channel and a longitudinally shiftable ring (2) having a small lever (3) is arranged at the tube-shaped handle (1), the ring (2) being positionable and clampable at the tube-shaped handle (1) in an arbitrary position.

2. A laryngoscope according to claim 1, **characterized in that** the bottom part is reinforced at the proximal end by increasing the wall thickness and/or applying a crimp.

## Revendications

1. Laryngoscope, pour l'examen de la cavité pharyngienne, avec une poignée tubulaire (1), une source lumineuse (6) et une spatule d'intubation présentant une partie inférieure pliée avec une baguette de guidage latéralement appliquée sur celle-ci, se rétrécissant à l'extrémité distale, ladite baguette de guidage présentant un rétrécissement à l'extrémité proximale, lequel s'étend sur le dernier tiers au moins de la longueur de la spatule jusqu'à une hauteur zéro, la branche horizontale s'étendant sur toute sa largeur jusqu'à l'extrémité proximale, la partie inférieure étant droite sur son extrémité proximale, **caractérisé en ce qu'**une lampe à lumière froide (6) et une canalisation sont disposées sur la spatule d'intubation, des fibres optiques se trouvant dans la canalisation, et **en ce qu'**une bague coulissant dans le sens de la longueur (2) avec un petit levier (3) est disposée sur la poignée tubulaire (1), laquelle est positionnable et serrable dans une position quelconque sur la poignée tubulaire.

2. Laryngoscope selon la revendication 1, **caractérisé en ce que** la partie inférieure sur l'extrémité proximale est raidie par agrandissement de l'épaisseur de paroi et/ou par application d'une moulure.
